# FASCICULE DE BREVET EUROPEEN

(11) **EP 2 478 351 B1**
(45) Date de publication et mention de la délivrance du brevet: **29.05.2013**
(21) Numéro de dépôt: 10773644.9
(22) Date de dépôt: 17.09.2010
(51) Int. Cl.: G01N 27/414, G01N 27/12

(54) **APPAREIL ET PROCEDE DE DETECTION ET/OU DE QUANTIFICATION DE COMPOSES D'INTERET PRESENTS SOUS FORME GAZEUSE OU EN SOLUTION DANS UN SOLVANT**
VORRICHTUNG UND VERFAHREN ZUR DETEKTION UND/ODER QUANTIFIZIERUNG VON IN GASFÖRMIGER FORM VORLIEGENDEN ODER IN EINEM LÖSUNGSMITTEL AUFGELÖSTEN RELEVANTEN VERBINDUNGEN
APPARATUS AND METHOD FOR DETECTING AND/OR QUANTIFYING COMPOUNDS OF INTEREST PRESENT IN GASEOUS FORM OR DISSOLVED IN A SOLVENT

(30) Priorité: 18.09.2009 FR 0904468
(43) Date de publication de la demande: 25.07.2012
(73) Titulaire: Commissariat à l'Énergie Atomique et aux Énergies Alternatives, 75015 Paris (FR)
(72) Inventeur: CARELLA, Alexandre, F-64110 Mazeres Lezons (FR); SIMONATO, Jean-Pierre, F-38360 Sassenage (FR)
(74) Mandataire: Noel, Chantal Odile
(86) Numéro de dépôt international: PCT/FR2010/000630
(87) Numéro de publication internationale: WO 2011/033195

(56) Documents cités:
- WO-A2-2006/099518
- ISHII A ET AL: "Ultrasensitive detection of organophosphate insecticides by carbon nanotube field-effect transistor", COLLOIDS AND SURFACES. A, PHYSICOCHEMICAL AND ENGINEERING ASPECTS, vol. 313-314, 27 décembre 2007 (2007-12-27), pages 456-460, XP022402311, ISSN: 0927-7757, DOI: 10.1016/j.colsurfa.2007.05.071
- DALE T J ET AL: "Fluorescent Sensors for Organophosphorous Nerve Agent Mimics", JOURNAL OF THE AMERICAN CHEMICAL SOCIETY, vol. 128, no. 14, 1 janvier 2006 (2006-01-01), pages 4500-4501, XP002518148, ISSN: 0002-7863, DOI: 10.1021/JA057449i cité dans la demande
- ZHANG S-W ET AL: "Fluorescent detection of chemical warfare agents: Functional group specific ratiometric chemosensors.", JOURNAL OF THE AMERICAN CHEMICAL SOCIETY, vol. 125, no. 12, 26 mars 2003 (2003-03-26), pages 3420-3421, XP002584897, ISSN: 0002-7863, DOI: 10.1021/JA029265z cité dans la demande
- ZHU M ET AL: "Visible near-infrared chemosensor for mercury ion", ORGANIC LETTERS, vol. 10, no. 7, avril 2008 (2008-04), pages 1481-1484, XP002584898, ISSN: 1523-7060, DOI: 10.1021/ol800197t cité dans la demande
- WANG F ET AL: "Carbon nanotube/polythiophene chemiresistive sensors for chemical warfare agents", JOURNAL OF THE AMERICAN CHEMICAL SOCIETY, vol. 130, no. 16, 23 avril 2008 (2008-04-23), pages 5392-5393, XP002555934, ISSN: 0002-7863, DOI: 10.1021/JA710795K [extrait le 2008-03-29]

## Description

L'invention se rapporte à un appareil et à un procédé de détection et/ou de quantification de composés d'intérêt présents sous forme gazeuse ou en solution dans un solvant.

De nombreux composés, qui peuvent se trouver sous forme gazeuse ou encore en solution dans un solvant, doivent pouvoir être détectés et/ou quantifiés rapidement, directement sur le site d'intervention, et de manière sélective.

De tels composés sont par exemple les composés organophosphorés qui sont des molécules constituées d'un atome de phosphore auxquels sont liés différents groupes chimiques dont la nature détermine les propriétés exactes du composé. Objet de recherches intensives sur les gaz de combat durant et après la seconde Guerre Mondiale qui ont abouti à la mise au point des gaz Sarin, Soman, Tabun, Cyclosarin, GV, VX, VE, VG et VM, et de molécules simulant l'action des organophosphorés neurotoxiques de type DFP (Diisopropylfluorophosphate), DCP (diéthylchlorophosphate) et DMMP (diméthyl-méthylphosphonate), les composés organophosphorés sont aujourd'hui principalement utilisés en agriculture comme insecticides ou herbicides. Parmi la grande variété de pesticides à base de composés organophosphorés existant on peut notamment citer le Parathion, le Malathion, le méthyl Parathion, le Chlorpyrifos, le Diazinon, le Dichlorvos, le Phosmet, le Tetrachlorvinphos et le méthyl Azinphos.

Le mode d'action des composés organophosphorés est basé sur l'affinité de ces derniers pour une enzyme impliquée dans la transmission de l'influx nerveux : la cholinestérase. Les composés organophosphorés ont en effet la propriété de se fixer de façon particulièrement forte et stable au niveau du site actif de cette enzyme. Une fois fixé, le composé organophosphoré empêche la cholinestérase de dégrader l'acétylcholine, neurotransmetteur libéré au niveau des synapses neuronales lors de l'excitation neuronale : faute de dégradation de l'acétylcholine en choline et acétyle inactifs, les neurones sont constamment excités, ce qui peut entraîner la paralysie du système nerveux central et conduire à la mort.

Le mécanisme impliqué dans la transmission de l'influx nerveux qui est bloqué par les composés organophosphorés étant identique dans l'ensemble du règne animal, les composés organophosphorés utilisés comme insecticides sont non seulement toxiques pour les insectes mais également pour tout animal, l'homme y compris. Pour cette raison, malgré leur relative bonne biodégradabilité qui leur a permis de supplanter les insecticides à base de composés organochlorés présentant une biodégradabilité faible, leur toxicité bien supérieure requiert des précautions d'emploi particulières. En effet, les composés organophosphorés peuvent poser de très sérieux problèmes du fait de l'accumulation dans l'environnement ou tout au long de la chaîne alimentaire en cas de présence de résidus sur les végétaux, dans l'eau, ou dans la viande des animaux ayant consommé des aliments contenant des composés organophosphorés. Ces insecticides étant parmi les plus utilisés non seulement dans l'agriculture par les professionnels mais aussi par les particuliers, la détection et le dosage des composés organophosphorés représentent un intérêt de santé publique et serait également particulièrement utile pour l'industrie agro-alimentaire.

En outre, malgré leur interdiction en 1997, les gaz de combat sont toujours une menace. D'une part la production des gaz de combat du type Sarin, Toman, VX est aisée, et d'autre part, ces composés étant inodores et incolores ils peuvent être inhalés sans que l'individu en prenne conscience. La détection immédiate de la présence de tels gaz est donc un enjeu majeur pour protéger les soldats en zone de combat mais aussi les populations civiles face au risque terroriste. Celui-ci est clairement avéré, notamment depuis l'attentat mortel au Sarin opéré par le groupe terroriste de Aum Shinrikyo dans le métro de Tokyo en 1995.

De nombreuses méthodes et dispositifs de détection des composés organophosphorés ont été développés.

Ainsi, des capteurs chimiques sensibles aux gaz organophosphorés, fonctionnant pour la plupart sur le principe des « nez électronique », sont commercialisés. Le Brevet US 5,571,401, par exemple, décrit un capteur chimique constitué de puces comprenant une résistance composée de polymères non-conducteurs et de matériaux conducteurs : lorsqu'une molécule chimique entre en contact avec les matériaux conducteurs, une différence de résistance est alors détectée. Ces capteurs ont l'inconvénient d'être peu spécifiques et d'être très sensibles aux faux positifs. Des capteurs à base de réseaux bidimensionnels de nanotubes de carbone ont également été mis au point (WO 2006/099518) mais, bien qu'ils soient très sensibles et détectent un grand nombre de molécules, ils ne permettent pas d'obtenir une réponse spécifique et sélective des composés organophosphorés.

Récemment des méthodes de détection sélectives des composés organophosphorés en solution basées sur la mesure de la fluorescence ont été décrites. Zhang *et al.* ont montré qu'en présence d'une molécule possédant une amine à proximité spatiale d'un alcool primaire et un chromophore non plan, flexible et faiblement conjugué, les composés organophosphorés réagissent avec l'alcool primaire, le phosphate obtenu permettant la cyclisation par substitution nucléophile intramoléculaire, stabilisant le chromophore dans le plan et aboutissant à une augmentation de la fluorescence (S.-W. Zhang and T. M. Swager, J.Am.Chem.Soc., 2003, 125, 3420-3421). Cette cyclisation permet donc une détection par fluorescence des agents organophosphorés en solution. L'équipe de J. Rebek, Jr. a ensuite montré que certains dérivés de l'acide de Kemp comportant un alcool primaire proche d'une amine tertiaire sont également de bons candidats pour détecter les espèces organophosphorées neurotoxiques (T. J. Dale & J. Rebek, Jr., J.Am.Chem.Soc., 2006, 4500-4501). Comme S.-W. Zhang *et al.* l'avait précédemment montré, lorsque l'alcool primaire réagit avec un composé organophosphoré, le phosphate obtenu permet la cyclisation par substitution nucléophile intramoléculaire et l'obtention d'un ammonium quaternaire selon la réaction présentée dans le schéma suivant :

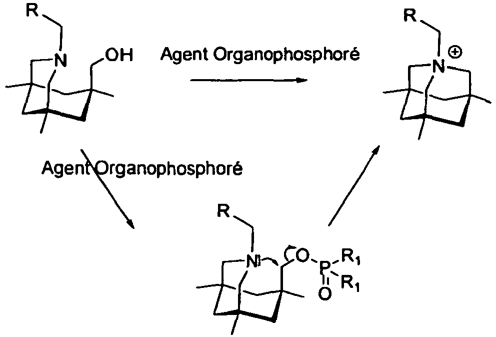

Rebek et coll. ont alors développé des dérivés de l'acide de Kemp où R est un fluorophore. Dans sa forme ouverte, la fluorescence est empêchée par un transfert d'électron photoinduit dû à l'amine. La cyclisation annule ce transfert d'électron et augmente la fluorescence de la molécule. Une exposition de 5 secondes d'un filtre comprenant l'acide de Kemp couplé à un fluorophore dans une atmosphère comportant 10 ppm de DFP permet la détection de cet organophosphoré par fluorescence par lecture sous lampe UV. Cette détection, bien que spécifique a divers inconvénients. Tout d'abord, elle nécessite d'être mise en oeuvre dans un environnement à luminosité réduite. Elle ne peut donc pas toujours être utilisée sur les lieux où la présence des composés organophosphorés est à détecter et en temps réel. D'autre part, elle nécessite l'emploi d'une lampe UV ce qui augmente l'encombrement, et donc diminue la portabilité, du dispositif de détection et/ou de quantification et des composés organophosphorés.

Des méthodes basées sur l'utilisation d'un biocapteur comprenant des enzymes présentant une affinité pour les composés organophosphorés, par exemple la cholinestérase, ont également été développées. Dans la demande PCT WO 2004/040004, des algues unicellulaires exprimant au niveau membranaire l'acétylcholinestérase ont été utilisées pour tester la présence de composés organophosphorés en milieu aqueux, le pourcentage d'inhibition de l'acétylcholinestérase étant corrélé avec la concentration en composés organophosphorés dans le liquide aqueux contrôlé. Cette méthode nécessite la mise en solution des composés organophosphorés avant leur détection.

Ishii *A. et al.,* décrivent un capteur à base de nanotubes de carbone pour détecter des insecticides organophosphorés.

Ce capteur comprend deux électrodes, un substrat revêtu, sur une face, d'un matériau isolant, matériau isolant sur lequel une molécule d'acétylcholinesterase qui détecte les composés organophosphorés est greffée et, sur l'autre face, d'un matériau semi-conducteur.

Un dispositif de mesure du courant circulant entre les deux électrodes permet de détecter la présence des composés organophosphorés.

Ce capteur comprend des parties "actives" sur chacune des faces.

Ainsi, sur une face, la partie active est la molécule qui détecte les composés organophosphorés, et sur l'autre face, la partie active est représentée par le dispositif électrique en lui-même, c'est-à-dire la couche de matériau semi-conducteur et les électrodes.

Dès lors, la fabrication de ce capteur et son intégration dans un dispositif sont délicates car il faut, lors de sa fabrication, retourner le substrat et protéger la face "activée" déjà générée, ce qui est frein à sa facilité de fabrication et engendre des sources de détérioration du capteur.

Cela implique également que lors de l'intégration de ces capteurs dans un dispositif plus complet, il faut prévoir de protéger et laisser disponible les deux faces du capteur.

Enfin, avec ce type de capteur, il n'est possible de détecter les composés organophosphorés qu'en solution.

La demande PCT WO 2006/099518 A2 décrit quant à elle, un capteur comprenant un substrat revêtu d'une couche d'oxyde thermique et d'un réseau de nanotubes et de deux électrodes ainsi que d'un dispositif pour mesurer la différence de capacitance entre les deux électrodes.

Ce document enseigne également que des molécules réceptrices permettant de détecter des composés organophosphorés peuvent être déposées sur la surface d'oxyde de silicium, après le dépôt des nanotubes et que ces molécules recouvrent partiellement les nanotubes.

Mais la sensibilité de ce type de capteur est limitée par le fait qu'on ne peut pas déposer ou greffer un nombre important de molécules sur la couche d'oxyde thermique, cette couche ne pouvant être activée en surface, avant le greffage des molécules réceptrices, car cette activation, qui est généralement effectuée par plasma, ozonolyse ou un traitement avec une solution contenant de l'acide fluorhydrique et de l'acide nitrique (solution piranha) endommagerait la couche de semi-conducteur, c'est-à-dire ici les nanotubes de carbone.

En résumé, les méthodes de détection des composés organophosphorés actuellement disponibles ont l'inconvénient soit de ne pas être sélectives des composés organophosphorés, soit d'être utilisables uniquement pour tester un échantillon en solution, soit de nécessiter un lecteur de fluorescence et un environnement à faible luminosité. Le développement d'une méthode rapide, efficace et spécifique, permettant la détection sélective de composés organophosphorés à la fois sous forme gazeuse et en solution, dans tout milieu, quelle que soit sa luminosité, ainsi que la mise au point d'un dispositif à encombrement réduit et bonne portabilité permettant une détection simple, rapide et sélective représentent donc toujours un enjeu majeur dans la protection des soldats en zone de combat, des populations civiles exposée au risque terroriste, et plus largement pour la détection de pesticides organophosphorés.

D'autres composés d'intérêt sont les ions mercuriques Hg²⁺.

Il est bien connu que le mercure est un composé toxique et/ou écotoxique sous toutes ses formes organiques et pour tous ses états chimiques.

En effet, le mercure s'accumule dans les organismes et est la cause de nombreuses maladies touchant spécialement les reins, le système digestif et le système neurologique.

De tous les degrés d'oxydation, ce sont les ions de mercure II, Hg²⁺, qui sont les plus toxiques.

La mise au point de capteurs sélectifs à cet élément est d'un intérêt particulier pour le dosage et la détection de cet élément dans le milieu naturel, l'eau et les aliments.

De plus, la détermination de la concentration en mercure dans les eaux destinées à l'alimentation est nécessaire dans le cadre des réglementations sur les eaux potables et sur les matières dangereuses.

La méthode couramment utilisée actuellement pour doser le mercure dans les eaux est la spectroscopie d'absorption atomique.

Cette méthode, bien que précise et fiable, a certains inconvénients.

En particulier, elle nécessite un équipement lourd difficilement transportable.

Les composés mercuriques peuvent également être détectés grâce à des capteurs colorimétriques et fluorescents sélectifs en greffant des chromophores ou des fluorophores sur des composés dithia dioxa-monoazaether couronnes.

Les composés dithia dioxa-monoazaether couronnes complexent sélectivement les ions Hg²⁺ et cette complexation entraîne une modification des propriétés des chromophores ou des fluorophores qui leurs sont liés.

Cette modification des propriétés optiques des chromophores ou des fluorophores est due à un effet électroattracteur du mercure qui appauvrit le chromophore.

Ainsi, Zhu et al. propose dans Org. Lett., 2008, 10, 1481-1484, un capteur chimique des ions mercuriques Hg²⁺ dans lequel un composé dithia dioxa-monoazaether couronne sur lequel est greffé un colorant tricarbocyanine est utilisé pour complexer les ions Hg²⁺ et ainsi provoque un changement de couleur du colorant lors de la complexation des ions Hg²⁺ par l'éther couronne.

Ce changement de couleur est visible à l'oeil nu.

Cependant, cette méthode ne permet pas la détection de faibles quantités d'ions Hg²⁺ et de plus ne permet pas de déterminer la concentration de Hg²⁺ dans l'échantillon analysé.

Dans ce même document, il est indiqué que la détection des ions mercuriques Hg²⁺ peut également être effectuée par analyse de la fluorescence émise par le colorant greffé sur l'éther couronne.

Cette méthode présente, outre l'impossibilité de déterminer la concentration en ions Hg²⁺, l'inconvénient de devoir être mise en oeuvre dans des conditions de luminosité réduite, ce qui ne lui permet pas d'être mise en oeuvre directement sur le site.

Le brevet US 7,385,267 B2 décrit des dispositifs électriques dans lesquels des nanotubes ou des nanofils, d'un matériau conducteur, fonctionnalisés par une molécule qui change de propriété au contact d'un analyte à détecter dans un échantillon.

Ce dispositif permet de détecter l'analyte dans l'échantillon par détection du changement de propriété du matériau conducteur.

Aucune référence n'est faite dans ce document à la détection d'ions Hg²⁺ et à la modification des propriétés de conduction d'un matériau semi-conducteur auquel est lié un composé complexant des ions mercuriques Hg²⁺ lors de son contact avec les ions Hg²⁺.

D'autres composés d'intérêt sont les composés nitrés, plus particulièrement les explosifs nitrés comme le TNT et les molécules basiques comme l'ammoniac.

Ainsi, il existe un besoin pour un appareil de détection et/ou de quantification de composés d'intérêt présents sous forme gazeuse dans l'air, par exemple, ou encore en solution dans l'eau ou dans un solvant inconnu, qui puisse être utilisé directement sur le site, quelques soient les conditions de luminosité, qui puisse être transportable, qui ait une grande sensibilité et qui puisse être fabriqué facilement et à grande échelle.

Pour répondre à ce besoin, l'invention propose d'utiliser la variation de charge induite lors de la mise en présence d'une molécule dite "réceptrice" comprenant un groupement réagissant avec les composés d'intérêt, soit par complexation soit par cyclisation ou toute autre réaction chimique, pour mettre en évidence la présence du, et quantifier le, composé d'intérêt dans l'échantillon à analyser.

Plus précisément, l'invention propose un appareil de détection et/ou de quantification sélective de composés d'intérêt présents sous forme gazeuse ou en solution dans un solvant comprenant :
- un dispositif électrique comprenant :
   ● deux électrodes,
   ● une couche de matériau diélectrique isolant,
   ● une couche comprenant une couche de molécule réceptrice comprenant au moins une molécule réceptrice A greffée sur la couche de matériau diélectrique isolant, ladite molécule réceptrice comprenant un groupement R₁ susceptible de réagir avec les composés d'intérêt, et
   ● une couche de matériau semi-conducteur déposée sur la couche de molécule réceptrice, et
- un dispositif de détection et/ou de mesure de la variation de charges positives entre les deux électrodes.

De préférence, la molécule réceptrice A comprend, de plus, un groupement R permettant son greffage sur la couche de matériau diélectrique isolant.

Dans un premier mode de réalisation de l'appareil de détection et/ou de quantification sélective de composés d'intérêt de l'invention, le matériau diélectrique isolant est choisi parmi les matériaux diélectriques isolants à base de silicium, les matériaux diélectriques isolants à base d'aluminium ou d'hafnium et les matériaux diélectriques isolants organiques.

Dans un second mode de réalisation plus particulièrement préféré de l'appareil de détection et/ou de quantification sélective de composés d'intérêt de l'invention, le matériau diélectrique isolant est choisi parmi l'oxyde de silicium, l'oxyde d'aluminium et le polyhexène diimide.

Dans un premier mode de réalisation préféré, le matériau diélectrique isolant est à base de silicium, de préférence est de l'oxyde de silicium (SiO₂), et le groupement R de la molécule réceptrice A est un groupement trihalogénosilane ou trialcoxy(en C₁ à C₄)silane, de préférence un groupement triméthoxysilane.

Dans un second mode de réalisation préféré, le matériau diélectrique isolant est à base d'aluminium, de préférence est de l'oxyde d'aluminium (Al₂O₃), et le groupement R de la molécule réceptrice A est un groupement trihalogénosilane ou trialcoxy(en C₁ à C₄)silane, de préférence un groupement triméthoxysilane.

Dans un troisième mode de réalisation préféré, le matériau diélectrique isolant est un matériau isolant diélectrique organique, de préférence est du polyhexène diimide, et le groupement R de la molécule réceptrice A est un groupement trihalogénosilane ou trialcoxy(en C₁ à C₄)silane, de préférence un groupement triméthoxysilane.

La molécule réceptrice A peut de plus comprendre une partie espaceur reliant le groupement R au groupement R₁, cette partie espaceur étant constituée d'une chaîne hydrocarbonée en C₁ à C₂₀ inclus, linéaire ou ramifiée, et pouvant contenir au moins un hétéroatome et/ou radical aromatique et/ou radical hétéroaromatique.

Dans un premier mode de mise en oeuvre, les composés d'intérêt sont des composés organophosphorés et le groupement R₁ de la molécule réceptrice A est un groupement constitué d'un groupe alcool primaire situé à proximité spatiale d'un groupe amine tertiaire.

Dans ce premier mode de mise en oeuvre, ladite molécule réceptrice A peut être une molécule obtenue à partir de l'acide de Kemp et a la formule générale (I) suivante : 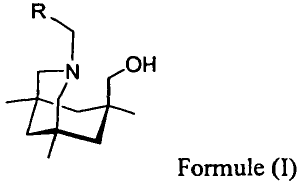 dans laquelle R représente le groupement de greffage, éventuellement muni de la partie espaceur.

Comme on le voit, dans cette formule (I), le groupement R₁ de la molécule réceptrice A est un groupement 1,5,7-triméthyl-3-azabicyclo[3.3.1]nonane-7-méthanol.

Mais, ladite molécule réceptrice A peut aussi être une molécule de formules générales (II) ou (III) suivantes : 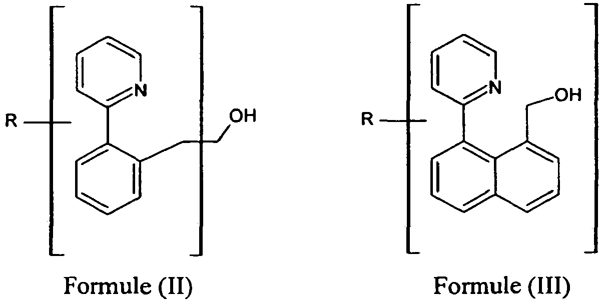 dans lesquelles R représente le groupement de greffage, éventuellement muni de la partie espaceur.

Dans ce cas, de préférence, le groupe R est un groupe triméthoxysilane.

Dans un second mode de mise en oeuvre de l'appareil de l'invention, les composés d'intérêt sont des ions mercuriques Hg²⁺, et le groupement R₁ de la molécule réceptrice A est choisi parmi un composé dithia dioxa-monoazaéther couronne, une N,N'(hydroxyéthyl) amine, une N,N'(carboxyléthyl) amine et les mélanges de deux au moins de ces composés.

De préférence, le groupement R₁ de la molécule réceptrice A est un groupement dithia dioxa-monoazaéther couronne.

Dans un troisième mode de mise en oeuvre de l'appareil de l'invention, les composés d'intérêt sont des composés nitrés et le groupement R₁ de la molécule réceptrice A est un groupement polyméthoxyarène, de préférence diméthoxybenzène.

Dans un quatrième mode de mise en oeuvre de l'appareil de l'invention, les composés d'intérêt sont des composés basiques et le groupement R₁ de la molécule réceptrice A est un groupement acide.

Dans tous les modes de réalisation et de mise en oeuvre de l'appareil de l'invention, le dispositif électrique peut être de type résistif.

Mais, le dispositif électrique peut aussi, et de préférence, être un transistor à effet de champ.

Quant à la couche de matériau semi-conducteur, de préférence, il s'agit d'une couche de nanotubes de carbone et/ou de nanofils à base de Si et/ou de feuillets de graphène.

L'invention propose également un procédé de détection et/ou de quantification de composés d'intérêt caractérisé en ce qu'il comprend les étapes suivantes :
a) mise en contact de l'échantillon susceptible de contenir les composés d'intérêt avec la au moins une molécule réceptrice A de l'appareil de détection et/ou de quantification, et
b) lecture de la variation de charge induite par la réaction de la molécule réceptrice A avec le composé d'intérêt en mesurant la différence de courant ou de tension entre les deux électrodes du dispositif électrique.

L'invention sera mieux comprise et d'autres caractéristiques et avantages de celle-ci apparaîtront plus clairement à la lecture de la description explicative qui suit.

L'appareil de détection et/ou de quantification sélective de composés d'intérêt présents dans un échantillon sous forme gazeuse ou encore en solution dans un solvant tel que l'eau ou tout autre solvant, comprend deux dispositifs qui peuvent être bien entendu combinés en un seul :
- un dispositif électrique comprenant deux électrodes séparées l'une de l'autre par une couche de matériau diélectrique isolant selon l'invention, au moins une molécule réceptrice A étant greffée sur cette couche de matériau diélectrique isolant, formant une couche de molécule réceptrice, qui est ensuite revêtue d'une couche d'un matériau semi-conducteur, et
- un dispositif de détection et/ou de mesure de la variation des charges positives entre les deux électrodes du dispositif électrique.

Ainsi, on a découvert que, de façon surprenante, alors que la molécule réceptrice A est revêtue d'une couche d'un matériau semi-conducteur, elle continue à réagir avec le composé d'intérêt à détecter et ce avec une grande sensibilité.

De plus, les molécules réceptrices A peuvent être greffées avec une forte densité surfacique, supérieure à 10¹³ molécules / cm² car il est possible d'activer la surface de matériau isolant, sans endommager la couche de matériau semi-conducteur, car ce dernier n'est pas encore présent, pour créer un grand nombre de sites réactifs pour le greffage des molécules réceptrices A.

Une telle activation de surface n'est pas possible lorsque les molécules réceptrices A sont à greffer ou déposer lorsque le matériau semi-conducteur est déjà présent.

De plus, cela évite toute réaction parasite entre le matériau semi-conducteur, lorsqu'il est déjà présent, ce matériau semi-conducteur possédant lui-même un certain nombre de défauts de surface, eux-mêmes réactifs avec les fonctions réactives (groupements R et R₁) des molécules réceptrices A.

Les molécules réceptrices A ne couvrent en aucun cas, ne serait-ce que partiellement, la couche de matériau semi-conducteur.

Le dispositif électrique peut être un dispositif électrique de type résistif tel qu'un transistor qui est un dispositif constitué d'un matériau conducteur placé entre deux électrodes, ou un dispositif de type transistor à effet de champ (FET).

Les transistors à effet de champ ont, et cela a déjà été démontré, d'excellentes propriétés de détection, notamment de gaz.

Parmi les transistors à effet de champ on peut citer les transistors à effet de champ à nanotubes de carbone (CNTFET) ou à nanofils de Si dans lesquels la couche de matériau semi-conducteur est une couche de nanotubes de carbone ou de nanofils de Si.

On peut également citer les transistors à effet de champs dans lesquels le matériau semi-conducteur est un matériau organique (OFET).

Quant aux électrodes du dispositif électrique, elles peuvent être métalliques, par exemple en or, en argent, en palladium, en platine, en titane, en silicium dopé, en cuivre, en nickel ou en siliciure.

Mais les électrodes peuvent également être en un matériau conducteur à base de nanotubes de carbone et/ou en poly(3,4-éthylène dioxythiophène)/poly(styrène sulfonate) (PEDOT/PSS).

Quant à la couche de matériau semi-conducteur, elle peut être en tout matériau semi-conducteur qui apparaîtra à l'homme de l'art.

De préférence, il s'agira d'un matériau en ou à base de carbone, de silicium, de germanium, de zinc, de gallium, d'indium, de cadmium, ou de mélanges de ceux-ci.

Dans l'invention, par les termes « matériau à base de X » on désigne un matériau comprenant au moins 20 % en moles de X par rapport au nombre total de moles présentes dans le matériau.

Le matériau semi-conducteur pourra être également un matériau semi-conducteur organique tels que des oligomères, des polymères ou des petites molécules d'un poids moléculaire en masse inférieur à 1000 g.mol⁻¹, tels que le pentacène.

Par exemple, les matériaux semi-conducteurs organiques peuvent être des matériaux à base de composés aromatiques hétérocycliques tels que le thiophène et le quaterthiophène et leurs dérivés tels que les P3HT (poly-3-hexylthiophènes) ; le pyrrole tel que le polypyrrole ; une arylamine telle que la triphénylamine et ses dérivés tels que les poly(triarylamine) ; de macrocycles hétérocycliques tels que les porphyrines telles que la tétraphénylporphyrine et les phtalocyanines et leurs dérivés tels que la tétraphénylporphyrine de cuivre et la phatocyanine de nickel ; des acènes polycycliques aromatiques tels que les pentacènes et leurs dérivés tels que le tri-isopropylsilyl-pentacène ; dés arylènes tels que le pyrène, et ses dérivés tels que le dicyanopérylènedümide (PDI-CN₂).

Des exemples de tels matériaux semi-conducteurs organiques préférés sont le poly-3-hexylthiophène, les poly(triarylamine), l'anthracène, le pentacène, le pérylène, le polyparaphénylène, le polyparaphénylènevinylène et le polyfluorène.

Cependant, le matériau semi-conducteur tout particulièrement préféré est constitué de nanofils et/ou de nanotubes de silicium et/ou de nanofils et/ou de nanotubes de carbone et/ou de nanofils et/ou de nanotubes d'un matériau à base de silicium et de germanium tel qu'un alliage de composition molaire Si_{0,7}Ge_{0,3}.

Quant au matériau diélectrique isolant, c'est un matériau isolant, avantageusement à base de silicium, comme par exemple l'oxyde de silicium, à base d'aluminium, comme par exemple, l'oxyde d'aluminium, ou à base d'un matériau organique tel que le polyhexene diimide.

La molécule réceptrice A est greffée sur le matériau diélectrique isolant du dispositif électrique.

A cet effet, de préférence, la molécule réceptrice A comprend un groupement R comprenant une fonction permettant le greffage de la molécule réceptrice A sur le matériau diélectrique isolant.

La fonction permettant le greffage, qui peut constituer le groupement R en entier, est adaptée au matériau diélectrique isolant. Le groupement R est généralement sélectionné parmi le groupement trihalogénosilane, trialcoxy (C₁ à C₄)silane, les acides carboxyliques et/ou sulfoniques et/ou phosphoriques.

Le greffage du groupement R peut conduire à la formation d'une monocouche auto-assemblée.

Ainsi, lorsque le matériau diélectrique isolant est à base de silicium, et plus particulièrement est de l'oxyde de silicium (SiO₂), le groupement R (ou plus précisément la fonction du groupement R) est un groupement trihalogénosilane ou trialcoxy (C₁ à C₄)silane, le plus préférablement un groupement thiméthoxysilane.

Lorsque le matériau diélectrique isolant est un matériau à base d'aluminium, et plus particulièrement est de l'oxyde d'aluminium (Al₂O₃), le groupement R comprend ou est un groupement trihalogénosilane ou trialcoxy(en C₁ à C₄)silane, de préférence un groupement triméthoxysilane.

Lorsque le matériau diélectrique isolant est un matériau diélectrique isolant organique, et plus particulièrement le polyhexène diimide, le groupement R comprend ou est constitué d'un groupe trihalogénosilane ou trialcoxy(en C₁ à C₄)silane, de préférence un groupement triméthoxysilane.

Quant au groupement susceptible de réagir avec les composés d'intérêt à détecter et/ou quantifier, c'est-à-dire le groupement R₁, ce groupement dépendra bien entendu de la nature des composés d'intérêt.

Les groupements de greffage R et le groupement susceptible de réagir avec le composé d'intérêt, c'est-à-dire le groupement R₁, peuvent être séparés l'un de l'autre par une partie espaceur.

Cette partie espaceur peut être constituée d'une chaîne hydrocarbonée en C₁ à C₂₀ inclus, linéaire ou ramifiée.

Par les termes « chaîne hydrocarbonée en C₁ à C₂₀ » on désigne, dans l'invention, une chaîne composée de 1 à 20 atomes de carbone liés entre eux par des liaisons saturées (liaisons simples) et/ou insaturées (liaisons doubles et/ou triples).

Elle peut contenir au moins un hétéroatome et/ou radical aromatique et/ou un radical hétéroatomique.

Le groupement R₁, lorsque les composés d'intérêt sont des molécules organophosphorées, est constitué d'un groupement comprenant un groupe alcool primaire situé à proximité spatiale d'un groupe amine tertiaire.

Dans ce cas, et dans une première variante, la molécule réceptrice A est un dérivé de l'acide de Kemp et a la formule générale (I) suivante : 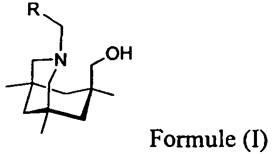 dans laquelle R représente le groupement de greffage ou comprenant une fonction de greffage de la molécule réceptrice, éventuellement séparé de l'atome d'azote par une partie espaceur.

Toujours dans le cas où les composés d'intérêt à détecter sont des composés organophosphorés, la molécule réceptrice A peut également être une molécule de formules générales (II) ou (III) suivantes :

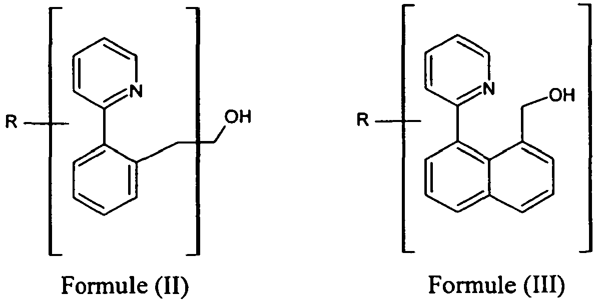

Une molécule réceptrice A de formule (I) particulièrement préférée dans l'invention est une molécule comprenant en tant que groupement R₁, un groupement 1,5,7-triméthyl-3-azabicyclo[3.3.1]nonane-7-méthanol, et plus particulièrement dans laquelle, en même temps, le groupement R de greffage est un groupement triméthoxysilane.

Avec les molécules réceptrice A de formule (I) ou de formule (II) ou (III), il se produit, lors de l'exposition de la molécule réceptrice A à un composé organophosphoré, la formation d'un intermédiaire phosphate ester instable par réaction entre l'alcool primaire et le composé organophosphoré.

Il s'ensuit une cyclisation intramoléculaire par substitution nucléophile de l'intermédiaire phosphate ester par l'amine tertiaire et la formation d'un ammonium quaternaire.

Lors de la réaction de cyclisation il y a formation d'un sel, et donc génération de charges électriques distinctes (cations et anions).

La génération d'une charge par la création de la fonction ammonium permet de modifier de façon brutale l'environnement électrostatique de la molécule et c'est cette modification qui est mesurée par le dispositif de détection et/ou de mesure de l'appareil de détection et/ou de quantification de l'invention, par exemple par mesure de variation du courant ou de la tension.

Lorsque les composés d'intérêt sont des ions mercuriques, la molécule réceptrice A comprend, outre le groupement de greffage sur le matériau diélectrique isolant, tel que défini ci-dessus, un groupement R₁ qui va permettre de complexer les ions mercuriques.

Ainsi, la molécule réceptrice A dans ce cas est une molécule dérivé d'un composé complexant les ions mercuriques Hg²⁺.

De tels composés préférés dans l'invention sont le dithia dioxa-monoazaéther couronne, une N,N'(hydroxyéthyl) amine, une N,N'(carboxyléthyl) amine et les mélanges de deux au moins de ces composés.

Un composé tout particulièrement préféré dans le cas de la détection et/ou de la quantification des ions mercuriques Hg²⁺ est le dithia dioxa-monoazaéther couronne.

Bien entendu, le groupement de greffage de la molécule réceptrice A dans ce cas peut également être lié au groupement R complexant les ions mercuriques Hg²⁺ par une partie espaceur, qui peut être la même que celle définie précédemment.

Lors de la complexation des ions mercuriques Hg²⁺, il se produit une variation du courant de conduction entre les deux électrodes du dispositif électrique selon l'invention et c'est cette variation du courant de conduction entre les deux électrodes qui est détecté et/ou mesuré par le dispositif de mesure de la variation de charge positive entre les deux électrodes selon l'invention.

Lorsque les composés d'intérêt sont des composés nitrés, tels que des explosifs nitrés, comme le TNT, le groupement R₁ est un groupe riche en électrons tel qu'un groupement polyméthoxyarène, par exemple un groupement diméthoxybenzène.

Lorsque les composés d'intérêt sont des molécules basiques telles que l'ammoniac, le groupement R₁ est un groupement acide, tel qu'un groupement acide carboxylique, sulfonique ou phosphorique, par exemple un groupement acide benzoïque.

L'appareil de détection et/ou de quantification de l'invention a une structure simple qui permet de le produire à bas coût et à grande échelle.

En effet, les deux parties actives de ce capteur, à savoir la partie purement électrique constituée des électrodes et du semi-conducteur et la couche de molécules réceptrices A, sont toutes deux sur la même face du substrat.

Il est donc très facile à fabriquer en ligne, contrairement au cas dans lequel les deux faces du substrat comprennent des parties actives.

Cela permet également que l'appareil de l'invention peut être de très petite taille, nécessitant peu d'énergie pour fonctionner, favorisant sa portabilité.

Afin de mieux faire comprendre l'invention, on va maintenant en décrire, à titre d'exemple purement illustratif et non limitatif un exemple de mise en oeuvre.

**EXEMPLE 1 :** Fabrication d'un appareil de détection et/ou de quantification d'un composé organophosphoré sous forme gazeuse, le diisopropylfluorophosphate (DFP).

Des dispositifs électriques ont été fabriqués sur un substrat de silicium recouvert d'une couche d'oxyde de silicium, comportant à sa surface, une couche d'oxyde de silicium d'une épaisseur de 100 nm.

Des électrodes en Ti/Au (5/30 nm) ont été déposées par évaporation sur la surface d'oxyde de silicium, qui constitue le matériau diélectrique isolant du dispositif électrique selon l'invention.

La surface de la couche d'oxyde de silicium est activée par un traitement plasma pour créer des fonctions hydroxyles de surface.

La surface est ensuite fonctionnalisée par immersion à température ambiante pendant 24 heures dans une solution 1mM de 11-azidoundecyltriméthoxysilane dans du toluène.

Le substrat est ensuite rincé abondamment avec du dichlorométhane, de l'eau et de l'acétone puis séché sous flux d'argon.

Puis, le groupement provoquant la cyclisation du composé organophosphoré a été greffé sur la monocouche d'azide dans du N,N'-diméthylformamide (DMF) à température ambiante en plongeant le substrat dans une solution à 2 mM de 1-(-4-éthynylbenzyl)-1,5,7-triméthyl-3-azabicyclo[3.3.1]nonane-7-méthanol contenant 1 % en mole de CuSO₄.5H₂O et 5% en mole de Na-ascorbate par rapport au composé alcyne.

Le substrat ainsi fonctionnalisé est ensuite soniqué successivement dans du DMF, de l'eau et du méthanol pendant 2 minutes puis séché sous flux d'argon.

Puis la couche de matériau semi-conducteur a été déposée sur la surface du matériau diélectrique isolant ainsi fonctionnalisé.

Cela a été fait par vaporisation d'une solution de nanotubes de carbone.

Le dispositif électrique est ensuite séché dans une étuve à vide à 100°C, 1mbar pendant 12 heures.

Ce dispositif est mis en présence de vapeur de diisopropylfluorophosphate et la variation de résistance a été mesurée.

On a mesuré une variation relative de la résistance ΔR/R de 42%.

Dans l'exemple 1 qui précède, le greffage de la molécule réceptrice A a été effectué en deux temps : tout d'abord la molécule comprenant une partie espaceur et le groupement de greffage triméthoxysilane a été greffé sur la couche de matériau diélectrique isolant puis le groupement de cyclisation R relié à une autre partie de la partie espaceur de la molécule réceptrice A, la partie 1-(-4-éthynylbenzyl) a été greffée sur la partie libre du groupement de greffage azido undécyle.

Cependant, le greffage de la molécule réceptrice A peut être effectué en une seule étape, sans s'écarter du cadre de l'invention.

**EXEMPLE 2:** Fabrication d'un appareil de détection et/ou de quantification d'un composé organophosphoré sous forme gazeuse, le diisopropylfluorophosphate (DFP).

Des dispositifs électriques ont été fabriqués sur un substrat de silicium recouvert d'une couche d'oxyde de silicium, comportant à sa surface, une couche d'oxyde de silicium d'une épaisseur de 100 nm.

Des électrodes en Ti/Au (5/30 nm) ont été déposées par évaporation sur la surface d'oxyde de silicium, qui constitue le matériau diélectrique isolant du dispositif électrique selon l'invention.

Puis la couche de matériau semi-conducteur a été déposée sur la surface du matériau diélectrique.

Cela a été fait par vaporisation d'une solution de nanotubes de carbone.

La surface encore accessible car non recouverte par les nanotubes de carbone est ensuite fonctionnalisée par immersion à température ambiante pendant 24 heures dans une solution 1mM de 11-azidoundecyltriméthoxysilane dans du toluène.

Le substrat est ensuite rincé abondamment avec du dichlorométhane, de l'eau et de l'acétone puis séché sous flux d'argon.

Puis, le groupement provoquant la cyclisation du composé organophosphoré a été greffé sur la monocouche d'azide dans du N,N'-diméthylformamide (DMF) à température ambiante en plongeant le substrat dans une solution à 2 mM de 1-(-4-éthynylbenzyl)-1,5,7-triméthyl-3-azabicyclo[3.3.1]nonane-7-méthanol contenant 1% en mole de CuSO₄.5H₂O et 5% en mole de Na-ascorbate par rapport au composé alcyne.

Le substrat ainsi fonctionnalisé est ensuite soniqué successivement dans du DMF, de l'eau et du méthanol pendant 2 minutes puis séché sous flux d'argon.

Le dispositif électrique est ensuite séché dans une étuve à vide à 100°C, 1mbar pendant 12 heures.

Ce dispositif est mis en présence de vapeur de diisopropylfluorophosphate et la variation de résistance a été mesurée.

On a mesuré une variation relative de la résistance ΔR/R de 17%.

Dans l'exemple 1 qui précède, le greffage de la molécule réceptrice A a été effectué en deux temps : tout d'abord la molécule comprenant une partie espaceur et le groupement de greffage triméthoxysilane a été greffé sur la couche de matériau diélectrique isolant puis le groupement de cyclisation R relié à une autre partie de la partie espaceur de la molécule réceptrice A, la partie 1-(-4-éthynylbenzyl) a été greffée sur la partie libre du groupement de greffage azido undécyle.

Cependant, le greffage de la molécule réceptrice A peut être effectué en une seule étape, sans s'écarter du cadre de l'invention.

**EXEMPLE 3 :** Fabrication d'un appareil de détection et/ou de quantification des ions mercuriques.

Des dispositifs électriques ont été fabriqués sur un substrat de silicium recouvert d'une couche d'oxyde de silicium, comportant à sa surface, une couche d'oxyde de silicium d'une épaisseur de 100 nm.

Des électrodes en Ti/Au (5/30 nm) ont été déposées par évaporation sur la surface d'oxyde de silicium, qui constitue le matériau diélectrique isolant du dispositif électrique selon l'invention.

La surface de la couche d'oxyde de silicium est activée par un traitement plasma pour créer des fonctions hydroxyles de surface.

La surface est ensuite fonctionnalisée par immersion à température ambiante pendant 24 heures dans une solution 1mM de 11-azidoundecyltriméthoxysilane dans du toluène.

Le substrat est ensuite rincé abondamment avec du dichlorométhane, de l'eau et de l'acétone puis séché sous flux d'argon.

Le groupement complexant le Hg²⁺ a été greffé sur la monocouche d'azoture dans du N,N'-diméthylformamide (DMF) à température ambiante en plongeant le substrat dans une solution à 2 mM de N-(-4-éthynylphényl)-dithia dioxa-monoazaéther couronne contenant 1% en mole de CuSO₄.5H₂O et 5% en mole de Na-ascorbate par rapport au composé alcyne

Le substrat ainsi fonctionnalisé est ensuite soniqué successivement dans du DMF, de l'eau et du méthanol pendant 2 minutes puis séché sous flux d'argon.

Puis la couche de matériau semi-conducteur a été déposée sur la surface du matériau diélectrique isolant ainsi fonctionnalisé.

Cela a été fait par vaporisation d'une solution de nanotubes de carbone.

Le dispositif électrique est ensuite séché dans une étuve à vide à 100°C, 1mbar pendant 12 heures.

Ce dispositif est mis en présence d'une solution 10 µM de perchlorate de mercure(II) pendant 5 minutes et la variation de résistance a été mesurée.

On a mesuré une variation relative de la résistance ΔR/R égale à 14 %.

Dans l'exemple 2 qui précède, le greffage de la molécule réceptrice A a été effectué en deux temps : tout d'abord la molécule comprenant une partie espaceur et le groupement de greffage triméthoxysilane a été greffé sur la couche de matériau diélectrique isolant puis le groupement de cyclisation R relié à une autre partie de la partie espaceur de la molécule réceptrice A, la partie 1-(-4-éthynylbenzyl) a été greffée sur la partie libre du groupement de greffage azido undécyle.

Cependant, le greffage de la molécule réceptrice A peut être effectué en une seule étape, sans s'écarter du cadre de l'invention.

EXEMPLE 4: Fabrication d'un appareil de détection et/ou de quantification des ions mercuriques.

Des dispositifs électriques ont été fabriqués sur un substrat de silicium recouvert d'une couche d'oxyde de silicium, comportant à sa surface, une couche d'oxyde de silicium d'une épaisseur de 100 nm.

Des électrodes en Ti/Au (5/30 nm) ont été déposées par évaporation sur la surface d'oxyde de silicium, qui constitue le matériau diélectrique isolant du dispositif électrique selon l'invention.

La couche de matériau semi-conducteur a ensuite été déposée sur la surface du matériau diélectrique isolant ainsi fonctionnalisé par vaporisation d'une solution de nanotubes de carbone.

La surface est ensuite fonctionnalisée par immersion à température ambiante pendant 24 heures dans une solution 1mM de 11-azidoundecyltriméthoxysilane dans du toluène.

Le substrat est ensuite rincé abondamment avec du dichlorométhane, de l'eau et de l'acétone puis séché sous flux d'argon.

Le groupement complexant le Hg²⁺ a été greffé sur la monocouche d'azoture dans du N,N'-diméthylformamide (DMF) à température ambiante en plongeant le substrat dans une solution à 2 mM de N-(-4-éthynylphényl)-dithia dioxa-monoazaéther couronne contenant 1% en mole de CuSO₄.5H₂O et 5% en mole de Na-ascorbate par rapport au composé alcyne

Le substrat ainsi fonctionnalisé est ensuite soniqué successivement dans du DMF, de l'eau et du méthanol pendant 2 minutes puis séché sous flux d'argon.

Le dispositif électrique est ensuite séché dans une étuve à vide à 100°C, 1mbar pendant 12 heures.

Ce dispositif est mis en présence d'une solution 10 µM de perchlorate de mercure(II) pendant 5 minutes et la variation de résistance a été mesurée.

On a mesuré une variation relative de la résistance ΔR/R de 7%.

Dans l'exemple 2 qui précède, le greffage de la molécule réceptrice A a été effectué en deux temps : tout d'abord la molécule comprenant une partie espaceur et le groupement de greffage triméthoxysilane a été greffé sur la couche de matériau diélectrique isolant puis le groupement de cyclisation R relié à une autre partie de la partie espaceur de la molécule réceptrice A, la partie 1-(-4-éthynylbenzyl) a été greffée sur la partie libre du groupement de greffage azido undécyle.

Cependant, le greffage de la molécule réceptrice A peut être effectué en une seule étape, sans s'écarter du cadre de l'invention.

Les couches formées des molécules réceptrices ont généralement et de préférence une épaisseur inférieure à 3 nm, ce qui est compatible avec une miniaturisation de l'appareil de l'invention.

Ces couches sont revêtues de la couche de matériau semi-conducteur et ne le recouvrent pas.

## Revendications

1. Appareil de détection et/ou de quantification sélective de composés d'intérêt présents sous forme gazeuse ou en solution dans un solvant comprenant :
- un dispositif électrique comprenant :
● deux électrodes,
● une couche de matériau diélectrique isolant,
● une couche comprenant une couche de molécule réceptrice comprenant au moins une molécule réceptrice A, ladite molécule réceptrice A comprenant un groupement R₁ susceptible de réagir avec les composés d'intérêt,
● une couche de matériau semi-conducteur, et
- un dispositif de détection et/ou de mesure de la variation de charges positives entre les deux électrodes,
**caractérisé en ce que** la couche de molécule réceptrice A est greffée sur la couche de matériau diélectrique isolant et revêtue de la couche de matériau semi-conducteur.

2. Appareil selon la revendication 1, **caractérisé en ce que** la au moins une molécule réceptrice A comprend de plus un groupement R permettant le greffage de la molécule réceptrice A sur le matériau diélectrique isolant.

3. Appareil selon la revendication 1 ou 2, **caractérisé en ce que** le matériau diélectrique isolant est choisi parmi les matériaux diélectriques isolants à base de silicium, les matériaux diélectriques isolants à base d'aluminium ou d'hafnium et les matériaux diélectriques isolants organiques, de préférence choisi parmi l'oxyde de silicium, l'oxyde d'aluminium et le polyhexène diimide.

4. Appareil selon la revendication 2 ou 3, **caractérisé en ce que** le matériau diélectrique isolant est à base de silicium, de préférence est de l'oxyde de silicium SiO₂, et **en ce que** le groupement R de la molécule réceptrice A est un groupement trihalogénosilane ou trialcoxy (en C₁ à C₄)silane, de préférence un groupement triméthoxysilane.

5. Appareil selon la revendication 2 ou 3, **caractérisé en ce que** le matériau diélectrique isolant est à base d'aluminium, de préférence est de l'oxyde d'aluminium Al₂O₃, et **en ce que** le groupement R de la molécule réceptrice A est un groupement trihalogénosilane ou trialcoxy (en C₁ à C₄)silane, de préférence un groupement triméthoxysilane.

6. Appareil selon la revendication 2 ou 3, **caractérisé en ce que** le matériau diélectrique isolant est un matériau isolant diélectrique organique, de préférence est du polyhexène diimide, et **en ce que** le groupement R de la molécule réceptrice A est un groupement trihalogénosilane ou trialcoxy (en C₁ à C₄)silane, de préférence un groupement triméthoxysilane.

7. Appareil selon l'une quelconque des revendications 2 à 6, **caractérisé en ce que** la molécule réceptrice A comprend de plus une partie espaceur reliant le groupement R au groupement R₁, cette partie espaceur étant constituée d'une chaîne hydrocarbonée en C₁ à C₂₀ inclus, linéaire ou ramifiée, et pouvant contenir au moins un hétéroatome et/ou radical aromatique et/ou radical hétéroaromatique.

8. Appareil selon l'une quelconque des revendications précédentes, **caractérisé en ce que** les composés d'intérêt sont des composés organophosphorés et **en ce que** le groupement R₁ de la molécule réceptrice A est un groupement constitué d'un groupe alcool primaire situé à proximité spatiale d'un groupe amine tertiaire, de préférence ladite molécule réceptrice A est obtenue à partir de l'acide de Kemp et a la formule générale (I) suivante : 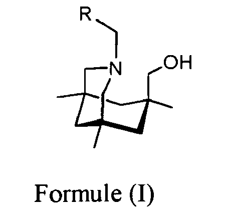 dans laquelle R représente le groupement de greffage, éventuellement muni de la partie espaceur.

9. Appareil selon la revendication 8, **caractérisé en ce que** ladite molécule réceptrice A est une molécule de formule générale (II) suivante : 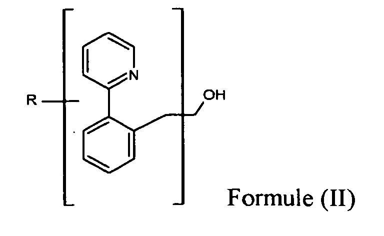 dans laquelle R représente le groupement de greffage, éventuellement muni de la partie espaceur.

10. Appareil selon la revendication 8, **caractérisé en ce que** ladite molécule réceptrice A est une molécule de formule générale (III) suivante : 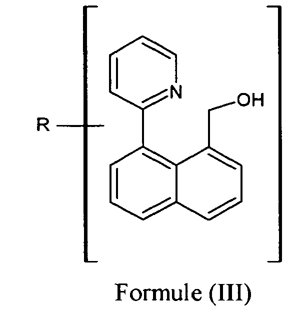 dans laquelle R représente le groupement de greffage, éventuellement muni de la partie espaceur.

11. Appareil selon l'une quelconque des revendications 8 à 10, **caractérisé en ce que** le groupement R est un groupe triméthoxysilane.

12. Appareil selon l'une quelconque des revendications 1 à 7, **caractérisé en ce que** les composés d'intérêt sont des ions mercuriques Hg²⁺, et **en ce que** le groupement R₁ de la molécule réceptrice A est choisi parmi un composé dithia dioxa-monoazaéther couronne, une N,N'(hydroxyéthyl) amine, une N,N'(carboxyléthyl) amine et les mélanges de deux au moins de ces composés, de préférence le groupement R₁ de la molécule réceptrice A est un groupement dithia dioxa-monoazaéther couronne.

13. Appareil selon l'une quelconque des revendications 1 à 7, **caractérisé en ce que** les composés d'intérêt sont des composés nitrés et **en ce que** le groupement R₁ de la molécule réceptrice A est un groupement polyméthoxyarène, de préférence diméthoxybenzène.

14. Appareil selon l'une quelconque des revendications 1 à 7, **caractérisé en ce que** les composés d'intérêt sont des composés basiques et **en ce que** le groupement R₁ de la molécule réceptrice A est un groupement acide.

15. Appareil selon l'une quelconque des revendications précédentes, **caractérisé en ce que** le dispositif électrique est de type résistif ou est un transistor à effet de champ.

16. Appareil selon l'une quelconque des revendications précédentes, **caractérisé en ce que** la couche de matériau semi-conducteur est une couche de nanotubes de carbone et/ou de nanofils à base de Si et/ou de feuillets de graphène.

17. Procédé de détection et/ou de quantification de composés d'intérêt **caractérisé en ce qu'**il comprend les étapes suivantes :
a) mise en contact de l'échantillon susceptible de contenir les composés d'intérêt avec la au moins une molécule réceptrice A de l'appareil de détection et/ou de quantification selon l'une quelconque des revendications 1 à 16, et
b) lecture de la variation de charge induite par la réaction de la molécule réceptrice A avec le composé d'intérêt en mesurant la différence de courant ou de tension entre les deux électrodes du dispositif électrique.

## Patentansprüche

1. Vorrichtung zur selektiven Detektion und/oder Quantifizierung von in gasförmiger Form oder in Lösung in einem Lösungsmittel vorliegenden Verbindungen von Interesse, umfassend:
- eine elektrische Vorrichtung, umfassend:
● zwei Elektroden,
● eine Schicht aus isolierendem dielektrischem Material,
● eine Schicht, die eine Rezeptormolekülschicht umfasst, die wenigstens ein Rezeptormolekül A umfasst, wobei das Rezeptormolekül A eine Gruppierung R₁ umfasst, die geeignet ist, mit den Verbindungen von Interesse zur reagieren,
● eine Schicht aus Halbleitermaterial, und
- eine Vorrichtung zur Detektion und/oder Messung der Änderung von positiven Ladungen zwischen den zwei Elektroden, **dadurch gekennzeichnet, dass** die Rezeptormolekülschicht A auf die Schicht aus isolierendem dielektrischem Material gepfropft ist und von der Schicht aus Halbleitermaterial überzogen ist.

2. Vorrichtung gemäß Anspruch 1, **dadurch gekennzeichnet, dass** das wenigstens eine Rezeptormolekül A darüber hinaus eine Gruppierung R umfasst, die die Pfropfung des Rezeptormoleküls A auf das isolierende dielektrische Material erlaubt.

3. Vorrichtung gemäß Anspruch 1 oder 2, **dadurch gekennzeichnet , dass** das isolierende dielektrische Material aus den isolierenden dielektrischen Materialien auf Siliciumbasis, den isolierenden dielektrischen Materialien auf Aluminium- oder Hafnium-Basis und den organischen isolierenden dielektrischen Materialien ausgewählt ist, vorzugsweise aus Siliciumoxid, Aluminiumoxid und Polyhexendiimid ausgewählt ist.

4. Vorrichtung gemäß Anspruch 2 oder 3, **dadurch gekennzeichnet, dass** das isolierende dielektrische Material auf Siliciumbasis ist, vorzugsweise Siliciumoxid SiO₂ ist, und dadurch, dass die Gruppierung R des Rezeptormoleküls A eine Trihalogensilan- oder Tri-(C₁-C₄)-alkoxysilan-Gruppierung ist, vorzugsweise eine Trimethoxysilan-Gruppierung ist.

5. Vorrichtung gemäß Anspruch 2 oder 3, **dadurch gekennzeichnet, dass** das isolierende dielektrische Material auf Aluminiumbasis ist, vorzugsweise das Aluminiumoxid Al₂O₃ ist, und dass die Gruppierung R des Rezeptormoleküls A eine Trihalogensilan- oder Tri-(C₁-C₄)-alkoxysilan-Gruppierung, vorzugsweise eine Trimethoxysilan-Gruppierung, ist.

6. Vorrichtung gemäß Anspruch 2 oder 3, dadurch g e **kennzeichnet,** dass das isolierende dielektrische Material ein organisches isolierendes dielektrisches Material ist, vorzugsweise Polyhexendiimid ist, und dass die Gruppierung R des Rezeptormoleküls A eine Trihalogensilan- oder Tri-(C₁₋C₄)-alkoxysilan-Gruppierung ist, vorzugsweise eine Trimethoxysilan-Gruppierung ist.

7. Vorrichtung gemäß einem der Ansprüche 2 bis 6, **dadurch gekennzeichnet,dass** das Rezeptormolekül A darüber hinaus einen Spacer-Teil umfasst, der die Gruppierung R mit der Gruppierung R₁ verbindet, wobei dieser Spacer-Teil aus einer Kohlenwasserstoffkette mit C₁-C₂₀, einschließlich, die linear oder verzweigt ist und wenigstens ein Heteroatom und/oder einen aromatischen Rest und/oder einen heteroaromatischen Rest enthalten kann, besteht.

8. Vorrichtung gemäß einem der vorangehenden Ansprüche, **dadurch gekennzeichnet,dass** die Verbindungen von Interesse Organophosphorverbindungen sind und dass die Gruppierung R₁ des Rezeptormoleküls A eine Gruppierung ist, die aus einer primären Alkoholgruppe besteht, die sich in räumlicher Nähe zu einer tertiären Amingruppe befindet, das genannte Rezeptormolekül A vorzugsweise ausgehend von Kemp-Säure erhalten wird und die folgende allgemeine Formel (I) hat: 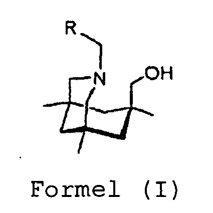 in der R die Pfropfungsgruppierung darstellt, die gegebenenfalls mit dem Spacer-Teil versehen ist.

9. Vorrichtung gemäß Anspruch 8, **dadurch gekennzeichnet, dass** das Rezeptormolekül A ein Molekül der folgenden allgemeinen Formel (II) ist: 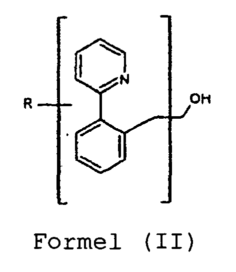 in der R die Pfropfungsgruppierung darstellt, die gegebenenfalls mit dem Spacer-Teil versehen ist.

10. Vorrichtung gemäß Anspruch 8, dadurch **gekenn** - **zeichnet**, dass das Rezeptormolekül A ein Molekül der folgenden allgemeinen Formel (III) ist: 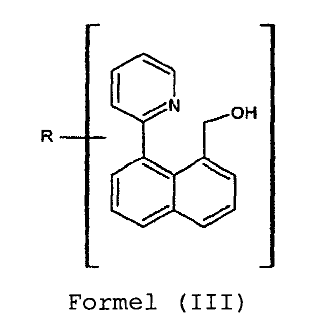 in der R die Pfropfungsgruppierung, gegebenenfalls versehen mit dem Spacer-Teil, darstellt.

11. Vorrichtung gemäß einem der Ansprüche 8 bis 10, **dadurch gekennzeichnet, dass** die Gruppierung R eine Trimethoxysilangruppe ist.

12. Vorrichtung gemäß einem der Ansprüche 1 bis 7, **dadurch gekennzeichnet, dass** die Verbindungen von Interesse Quecksilberionen Hg²⁺ sind und dass die Gruppierung R₁ des Rezeptormoleküls A ausgewählt ist aus einer Dithia-dioxa-monoazaether-Krone-Verbindung, einem N,N'-(Hydroxyethyl)-amin, einem N,N'-(Carboxylethyl)-amin und Gemischen von wenigstens zwei dieser Verbindungen, wobei die Gruppierung R₁ des Rezeptormoleküls A vorzugsweise eine Dithia-dioxa-monoazaether-Krone-Gruppierung ist.

13. Vorrichtung gemäß einem der Ansprüche 1 bis 7, **dadurch gekennzeichnet, dass** die Verbindungen von Interesse nitrierte Verbindungen sind und dass die Gruppierung R₁ des Rezeptormoleküls A eine Polymethoxyaren-Gruppierung, vorzugsweise Dimethoxybenzol, ist.

14. Vorrichtung gemäß einem der Ansprüche 1 bis 7, **dadurch gekennzeichnet, dass** die Verbindungen von Interesse basische Verbindungen sind und dass die Gruppierung R₁ des Rezeptormoleküls A eine saure Gruppierung ist.

15. Vorrichtung gemäß einem der vorangehenden Ansprüche, **dadurch gekennzeichnet, dass** die elektrische Vorrichtung vom resistiven Typ ist oder ein Feldeffekttransistor ist.

16. Vorrichtung gemäß einem der vorangehenden Ansprüche, **dadurch gekennzeichnet, dass** die Schicht aus Halbleitermaterial eine Schicht aus Kohlenstoffnanoröhren und/oder Nanofäden auf Si-Basis und/oder aus Graphenplättchen ist.

17. Verfahren zur Detektion und/oder Quantifizierung von Verbindungen von Interesse, **dadurch gekennzeichnet, dass** es die folgenden Stufen umfasst:
a) Inkontaktbringen der Probe, die die Verbindungen von Interesse enthalten kann, mit dem wenigstens einen Rezeptormolekül A der Vorrichtung zur Detektion und/oder Quantifizierung gemäß einem der Ansprüche 1 bis 16 und
b) Ablesen der Ladungsänderung, die durch die Reaktion des Rezeptormoleküls A mit der Verbindung von Interesse induziert wurde, indem die Strom- oder Spannungsdifferenz zwischen den zwei Elektroden der elektrischen Vorrichtung gemessen wird.

## Claims

1. Apparatus for selectively detecting and/or quantifying compounds of interest present in gaseous form or dissolved in a solvent, comprising:
- an electrical device comprising:
● two electrodes,
● a layer of dielectric insulating material,
● a layer comprising a layer of receptor molecule comprising at least one receptor molecule A, said receptor molecule A comprising a group R₁ capable of reacting with the compounds of interest,
● a layer of semiconductor material, and
- a device for detecting and/or measuring the variation in positive charges between the two electrodes,
**characterised in that** the layer of receptor molecule A is grafted onto the layer of dielectric insulating material and coated with the layer of semiconductor material.

2. Apparatus as claimed in claim 1, **characterised in that** the at least one receptor molecule A further comprises a group R enabling the receptor molecule A to be grafted onto the dielectric insulating material.

3. Apparatus as claimed in claim 1 or 2, **characterised in that** the dielectric insulating material is selected from dielectric insulating materials with a base of silicon, dielectric insulating materials with a base of aluminium or hafnium and organic dielectric insulating materials, preferably chosen from silicon oxide, aluminium oxide and polyhexene diimide.

4. Apparatus as claimed in claim 2 or 3, **characterised in that** the dielectric insulating material is based on silicon, and is preferably silicon oxide SiO₂ and the group R of the receptor molecule A is a trihalogenosilane or trialcoxy (C₁ to C₄) silane group, preferably a trimethoxysilane group.

5. Apparatus as claimed in claim 2 or 3, **characterised in that** the dielectric insulating material is based on aluminium, and is preferably aluminium oxide Al₂O₃, and the group R of the receptor molecule A is a trihalogenosilane or trialcoxy (C₁ to C₄) silane group, preferably a trimethoxysilane group.

6. Apparatus as claimed in claim 2 or 3, **characterised in that** the dielectric insulating material is an organic dielectric insulating material, and is preferably polyhexene diimide, and the group R of the receptor molecule A is a trihalogenosilane or trialcoxy (C₁ to C₄) silane group, preferably a trimethoxysilane group.

7. Apparatus as claimed in any one of claims 2 to 6, **characterised in that** the receptor molecule A further comprises a spacer part linking the group R to the group R₁, this spacer part being made up of a linear or branched C₁ to C₂₀ inclusive hydrocarbon chain, possibly containing at least one heteroatom and/or aromatic radical and/or heteroatomic radical.

8. Apparatus as claimed in any one of the preceding claims, **characterised in that** the compounds of interest are organophosphorous compounds and the group R₁ of the receptor molecule A is a group made up of a primary alcohol group disposed in spatial proximity to a tertiary amine group, said receptor molecule A preferably being obtained from Kemp=s acid and having the following general formula (I): 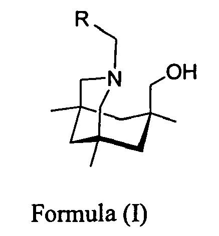 in which R represents the grafting group, optionally incorporating the spacer part.

9. Apparatus as claimed in claim 8, **characterised in that** said receptor molecule A is a molecule of the following general formula (II): 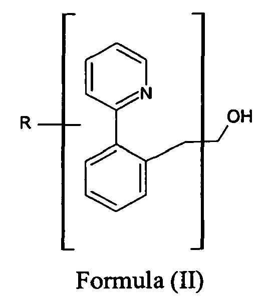 in which R represents the grafting group, optionally incorporating the spacer part.

10. Apparatus as claimed in claim 8, **characterised in that** said receptor molecule A is a molecule of the following general formula (III): 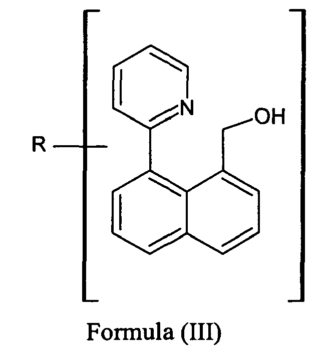 in which R represents the grafting group, optionally incorporating the spacer part.

11. Apparatus as claimed in any one of claims 8 to 10, **characterised in that** the group R is a trimethoxysilane group.

12. Apparatus as claimed in any one of claims 1 to 7, **characterised in that** the group R₁ of the receptor molecule A is selected from a dithia-dioxa-monoaza crown ether compound, an N,N=(hydroxyethyl) amine, an N,N=(carboxyethyl) amine and mixtures of at least two of these compounds, the group R₁ of the receptor molecule A preferably being a dithia-dioxa-monoaza crown ether group.

13. Apparatus as claimed in any one of claims 1 to 7, **characterised in that** the compounds of interest are compounds containing nitrogen and the group R₁ of the receptor molecule A is a polymethoxyarene group, preferably dimethoxybenzene.

14. Apparatus as claimed in any one of claims 1 to 7, **characterised in that** the compounds of interest are basic compounds and the group R₁ of the receptor molecule A is an acid group.

15. Apparatus as claimed in any one of the preceding claims, **characterised in that** the electrical device is of the resistive type or is a field-effect transistor.

16. Apparatus as claimed in any one of the preceding claims, **characterised in that** the layer of semiconductor material is a layer of carbon nanotubes and/or nanowires with an Si base and/or graphene sheets.

17. Method of detecting and/or quantifying compounds of interest, **characterised in that** it comprises the following steps:
a) placing the sample likely to contain the compounds of interest in contact with at least one receptor molecule A of the detection and/or quantification apparatus as claimed in any one of claims 1 to 16, and
b) reading the variation in charged induced by the reaction of the receptor molecule A with the compound of interest by measuring the difference in current or voltage between the two electrodes of the electrical device.
